# EUROPEAN PATENT SPECIFICATION

(11) **EP 4 669 400 B1**
(45) Date of publication and mention of the grant of the patent: **08.07.2026**
(21) Application number: 23821383.9
(22) Date of filing: 04.12.2023
(51) Int. Cl.: A61M 25/00

(54) **A CATHETER FOR DELIVERING MEDICAL THERAPY TO A REMOTE SITE IN A BODY**
KATHETER ZUR VERABREICHUNG EINER MEDIZINISCHEN THERAPIE AN EINE ENTFERNTE STELLE IN EINEM KÖRPER
CATHÉTER POUR ADMINISTRER UNE THÉRAPIE MÉDICALE À UN SITE DISTANT DANS UN CORPS

(30) Priority: 29.03.2023 EP 23165036
(43) Date of publication of application: 31.12.2025
(73) Proprietor: IMDS R&D B.V., 9301 NZ Roden (NL)
(72) Inventor: SCHULTING, Edwin Alexander, 9301 NZ Roden (NL); LEIBUNDGUT, Gregor, 9301 NZ Roden (NL)
(74) Representative: V.O.
(86) International application number: PCT/NL2023/050637
(87) International publication number: WO 2024/205394

(56) References cited:
- US-A1- 2011 112 476
- US-A1- 2016 015 928
- US-A1- 2019 255 290
- US-A1- 2021 307 766

## Description

The invention relates to a catheter for delivering medical therapy to a remote site in a body.

In said medical therapy, such as for example an interventional cardiovascular or neurovascular application, such a catheter must be angled through the tortuous bends and curves of a (vasculature) passageway to arrive at the targeted anatomy. Such a catheter requires sufficient flexibility, particularly closer to its distal end, to navigate such tortuous pathways. However, other design aspects must also be considered. For example, the catheter must also be able to provide sufficient torquability (i.e., the ability to transmit torque applied at the proximal end all the way to the distal end), pushability (i.e., the ability to transmit axial push to the distal end), and structural integrity for performing intended medical functions.

US 2021/0023339 A1 discloses that a kink-resistance catheter has at least a first pair of core wires residing in the catheter body on opposed sides of a catheter lumen. The core wires have relatively stiff proximal ends but taper toward their distal ends. The tapered construction provides the core wires with a degree of distal flexibility that helps the catheter advance along a vasculature to a site of interest without kinking. Moreover, the core wires greatly improve the torsional rigidity of the catheter so that rotation of the catheter's proximal end translates into a substantially equivalent rotation at the distal end. If desired, shaping ribbons are provided in the catheter body adjacent to the distal ends of the core wires. See, for example, the shaping ribbons 160 and 162 on the right-hand side of Fig. 5 of US 2021/0023339 A1. The shaping ribbons can be pre-bent by a physician prior to a surgical procedure to help the physician advance the catheter along the vasculature. That is, the shaping ribbons can be pre-bent by the physician into a desired deflection angle and to hold the angle. This is helpful when the catheter is intended to be used in a medical procedure where the vasculature leading to the target site has a generally known approach angle. Finally, the core wires provide the ability to push the catheter through the vasculature without the need for the catheter to go over a guidewire already in-situ in the vasculature.

It is further noted that US 2011/112476 A1 discloses a catheter according to the pre-characterizing portion of the appended independent claim 1 of the present disclosure.

It is the object of the present invention to provide an alternative catheter having a 'shapeable' catheter section close to the distal end, which is pre-bendable by a physician with well-balanced omni-directional bendability and with high shape retention, while allowing the catheter to be reliably made in an efficient manner, and to be designed with favourable properties with regard to flexibility, torquability, pushability and integrity of lumen structure.

For that purpose the invention provides a catheter according to the appended independent claim 1. Preferable embodiments of the invention are provided by the appended dependent claims 2-9.

Hence, the invention provides a catheter for delivering medical therapy to a remote site in a body, wherein:
the catheter has a proximal end, a distal end and a longitudinal direction extending from the proximal end to the distal end;
the catheter comprises a tubular catheter wall, which is surrounding a lumen structure of the catheter, and which is extending from the distal end, proximally along the longitudinal direction, to at least 100 mm proximal from the distal end;
the catheter wall comprises a pre-bendable wall section, which is manually pre-bendable all along a pre-bending range along the longitudinal direction, wherein said pre-bending range is at least extending between 5 mm proximal from the distal end and 10 mm proximal from the distal end;
the pre-bendable wall section comprises:
   - a pre-bendable skeleton, which is extending at least all along said pre-bending range, and which provides reinforcement of the catheter wall in the longitudinal direction, as well in a circumferential direction around the longitudinal direction, wherein the pre-bendable skeleton is an interrupted tubular reinforcement wall obtained by microfabricating a cut pattern in a fully uninterrupted tubular reinforcement wall made of a reinforcement material,
   - an inner liner, which is co-axially surrounded by said pre-bendable skeleton relative to a center line of the catheter wall, and
   - an outer laminate, which is co-axially surrounding said pre-bendable skeleton relative to the center line of the catheter wall;
the pre-bendable skeleton comprises:
   - a number N of right-handed helical struts, which are extending along N right-handed helical directions, respectively, wherein N ≥ 2,
   - N left-handed helical struts, which are extending along N left-handed helical directions, respectively, and
   - a plurality of ring-shaped struts, which are extending along circular directions, respectively;
each of said right-handed helical struts, left-handed helical struts and ring-shaped struts are co-axially arranged relative to the center line, wherein the N right-handed helical struts are angularly equally spaced relative to one another by 360 divided by N degrees around the center line, wherein the N left-handed helical struts are angularly equally spaced relative to one another by 360 divided by N degrees around the center line, wherein the ring-shaped struts are extending mutually parallel relative to one another and mutually spaced apart in the longitudinal direction, wherein the pre-bendable skeleton comprises for each of said ring-shaped struts N connection nodes at N angularly equally spaced positions, respectively, of each ring-shaped strut concerned, and wherein at each connection node, each ring-shaped strut concerned is connected to one of the N right-handed helical struts as well as to one of the N left-handed helical struts;
an overall skeleton cut-away ratio of a longitudinal section of the catheter wall in a longitudinal range along the longitudinal direction is defined as the percentage of:
   - the volume of all parts, within said longitudinal range, of said reinforcement material being cut-away from said fully uninterrupted tubular reinforcement wall according to said microfabricated cut pattern,
relative to
   - the volume of all parts, within said longitudinal range, of said reinforcement material of said fully uninterrupted tubular reinforcement wall; and
the overall skeleton cut-away ratio of the pre-bendable wall section in said pre-bending range is between 45% and 85%.

In the above-recited features of the present invention, the key features of the invention are that:
i. the pre-bendable wall section comprises the pre-bendable skeleton obtained by microfabricating said cut pattern in such manner that the pre-bendable skeleton comprises the recited specific structure of the N right-handed helical struts, the N left-handed helical struts, the ring-shaped struts and the connection nodes; and
ii. the overall skeleton cut-away ratio of the pre-bendable wall section is between 45% and 85%.

Said key feature (i) relates to the specific structure of the pre-bendable skeleton. Therein, the parallel ring-shaped struts provide hoop strength without hampering the pre-bendability of the pre-bendable wall section. At the same time the oppositely directed two sets of N equally spaced helical struts provide shape retention, as well as a well-balanced omni-directional bendability under the pre-bending action by a physician. Said pre-bendability is guided and regulated by the positions of the connection nodes, as well as by the parallel ring-shaped struts. Once being bent into a desired shape, the shape retention of the bent catheter section remains intact, since the pre-bendable skeleton is a triangular structure formed by nothing but triangles. That is, all connection nodes of the pre-bendable skeleton are vertices of multiple triangles, in which each triangle is formed by three strut sections of a left-handed helical strut, a right-handed helical strut and a ring-shaped strut, respectively. Such a triangular structure is strong.

Said key feature (ii) relates to the overall skeleton cut-away ratio of the pre-bendable wall section. Said cut-away ratio being not lower than 45%, as according to the lower limit specified by key feature (ii), prevents a too cumbersome pre-bendability of the wall section for a physician. The overall skeleton cut-away ratio of the pre-bendable wall section being not higher than 85%, as according to the upper limit specified by key feature (ii), prevents a troublesome shape retention after bending of the wall section.

Furthermore, it will be readily appreciated that the pre-bendable skeleton provides the pre-bendable wall section with very good torquability, pushability and integrity of lumen structure. Other longitudinal sections of the catheter can be designed in many various ways and be connected firmly to, or integrally with, the pre-bendable wall section having the pre-bendable skeleton. Hence, the invention allows the catheter to be reliably made in an efficient manner, and to be designed with favourable properties with regard to flexibility, torquability, pushability and integrity of lumen structure.

It is noted that manufacturing said high overall skeleton cut-away ratio (at least 45%) of the pre-bendable wall section according to the invention requires inventive measures to solve several problems. The reason is that the inner liner of a catheter having a skeleton for many applications typically is a tube made of, for example, PTFE (polytetrafluoroethylene, e.g. Teflon^{®}), PVDF (polyvinylidene fluoride, e.g. Kynar^{®}) or HDPE (high-density polyethylene), which during manufacturing of the catheter is axially inserted into the skeleton, and which is then expanded against the skeleton by inflation and heat. For said high cut-away ratios the problems arise that the tube made of, for example, PTFE, PVDF or HDPE being inflated against the skeleton will bulge out of the large cut-outs in the skeleton material and/or will break under the inflation pressure. Another problem, especially for skeletons made of less strong material, is that the skeleton may severely deform and/or crack and/or break under the inflation pressure due to the fact that the skeleton is severely weakened by the large cut-outs in the skeleton material.

In connection therewith, the present invention inter alia is based on the new insights, as disclosed herein, that said problems can be solved by introducing, temporarily during the manufacturing of a catheter according to the invention, a heat-shrinkable outer tube over the outer side of the pre-bendable skeleton, so that the heat-shrinkable outer tube temporarily closes off the cut-outs in the skeleton material during said expansion of the inner liner by inflation and heat, while at the same time the heat-shrinkable outer tube temporarily reinforces the pre-bendable skeleton during said expansion of the inner liner by inflation and heat. After the inner liner has been successfully expanded against the skeleton and after the catheter under construction has been cooled, the temporary heat-shrinkable outer tube can be removed from the outer side of the skeleton, and be replaced by the final outer laminate over the outer side of the skeleton.

Alternatively, another new insight, as disclosed herein, is that said problems can be solved by performing the following manufacturing steps for assembling the inner liner and the outer laminate to the skeleton in case of said high overall skeleton cut-away ratio (at least 45%) of the pre-bendable wall section of the catheter wall of a catheter according to the invention. Firstly, a tubular base layer of fluorinated ethylene propylene (FEP) is extruded and the inner liner is extruded over the base layer. Next, the FEP base layer together with the inner liner is axially inserted into the skeleton. Next, the outer laminate is slided over the outer side of the skeleton. Thereafter, a temporary heat-shrinkable outer FEP tube is slided over the outer laminate, and the total assembly is heated. During heating the temporary heat-shrinkable outer FEP tube provides high compression onto the assembly of the inner liner, the skeleton and the outer laminate. After the catheter under construction has been cooled, the temporary heat-shrinkable outer tube can be removed from the outer side of the skeleton, and the FEP base layer can be axially pulled out of the assembly, making use of the favourable properties of FEP with respect to being pulled. Thus, a very close fit of the inner liner against the inner side of the skeleton is obtainable.

It is noted that the last-mentioned use of such an extruded FEP base layer is a more effective and much less expensive alternative as compared to the traditional use of a temporary silver plated copper wire, which at the end of a manufacturing sub-process is axially pulled out of an inner liner of a catheter under construction.

Another advantage of the present invention is that, thanks to the high overall skeleton cut-away ratio (at least 45%) of the pre-bendable wall section of the catheter wall of a catheter according to the invention, and thanks to the above-mentioned insights as disclosed herein, the invention allows for efficiently embedding a radiopaque element (e.g. a radiopaque marker) in the catheter wall, since the radiopaque element, can be perfectly located in a space of the cut pattern, said space being bounded by the pre-bendable skeleton, the inner layer and the outer laminate.

In a preferable embodiment of a catheter according to the invention, said pre-bending range is at least extending between 0.5 mm proximal from the distal end and 15 mm proximal from the distal end. Thus the pre-bendable skeleton is distally extending yet closer to the distal end, which increases the physician's options to shape the catheter close to the distal end.

More preferably, said pre-bending range is at least extending between the distal end and 20 mm proximal from the distal end, which increases the physician's options to shape the catheter yet further.

In another preferable embodiment of a catheter according to the invention, the overall skeleton cut-away ratio of the pre-bendable wall section in said pre-bending range is between 60% and 80%. This further eases the shapeability of the wall section for a physician and further improves the shape retention after shaping.

More preferably, the overall skeleton cut-away ratio of the pre-bendable wall section in said pre-bending range is between 70% and 78%. This yet further eases the shapeability of the wall section for a physician and yet further improves the shape retention after shaping.

In another preferable embodiment of a catheter according to the invention, a radiopaque element is embedded in the catheter wall in that the radiopaque element is located in a space of the cut pattern, said space being bounded by the pre-bendable skeleton, the inner layer and the outer laminate.

The catheter according to the invention may be embodied as a rapid exchange catheter having the tubular catheter wall as a distal rapid exchange tubular segment which is spaced from the proximal end of the rapid exchange catheter.

Such a rapid exchange catheter has a proximal catheter segment, proximally from the distal rapid exchange tubular segment, which may, for example, be a push rod which is connected to the distal rapid exchange tubular segment. Such a push rod may advantageously have a round design with a diameter ≤ 0.40 mm to support 1:1 torquability from the proximal end to the distal end. Such a 1:1 torquability is highly desirable for this kind of device as the physician needs to be able to precisely steer the catheter towards a target site, such as a bend or a side branch of a vasculature passageway.

Alternatively, the catheter according to the invention may be embodied as an over-the-wire (OTW) catheter, wherein the tubular catheter wall and the lumen structure of the catheter are extending all along the longitudinal direction from the proximal end to the distal end.

Such an over-the-wire catheter may advantageously have an over-the-wire catheter skeleton, which is extending all along the longitudinal direction from the proximal end to the distal end, and which provides reinforcement of the catheter wall in the longitudinal direction, as well in the circumferential direction around the longitudinal direction, wherein the over-the-wire catheter skeleton is an interrupted tubular reinforcement wall obtained by microfabricating a cut pattern in a fully uninterrupted tubular reinforcement wall made of a reinforcement material. Hence, in this case, the pre-bendable skeleton of the pre-bendable wall section is a section of the over-the-wire catheter skeleton. Preferably the over-the-wire catheter skeleton is designed to support 1:1 torquability from the proximal end to the distal end of the over-the-wire catheter. Alternatively to said over-the wire skeleton, the over-the-wire catheter may, in longitudinal sections outside the pre-bending range of the pre-bendable skeleton, also be designed with, for example, braids or wire coil structures to support the 1:1 torquability.

In another preferable embodiment of a catheter according to the invention, the N right-handed helical struts and the N left-handed helical struts each have the same pitch as compared to one another, said pitch being defined as the distance, measured along the longitudinal direction, of one helical winding of a helical strut concerned, and wherein the pre-bending range has at least one continuous sub-range having a length equal to said pitch, and wherein the pre-bendable skeleton has a number of N multiplied by 2 of said ring-shaped struts in said continuous sub-range.

In the following, the invention is further elucidated with reference to a non-limiting embodiment and with reference to the schematic figures in the appended drawing, in which the following is shown.
Fig. 1 shows, in a longitudinal side view, an example of an embodiment of a catheter according to the invention, wherein in Fig. 1 the pre-bending range of the pre-bendable wall section of the catheter wall has been indicated by a brace mark, and wherein in the embodiment of the catheter of Fig. 1 the above-mentioned number N is equal to two (N = 2).
Fig. 2 shows, in a perspective view, a longitudinal section of the pre-bendable skeleton of the pre-bendable wall section of the catheter of Fig. 1.
Fig. 3A shows a short longitudinal section of the pre-bendable wall section of the catheter of Fig. 1, wherein the shown longitudinal section comprises a part of the pre-bendable skeleton, and wherein the shown view is a longitudinal sectional view, which contains the center line of the catheter wall.
Fig. 3B shows a cross-section through the longitudinal section of the pre-bendable wall section of Fig. 3A, wherein the shown cross-section is perpendicular to the center line of the catheter wall.
Fig. 4 shows a short longitudinal section of the pre-bendable skeleton of the pre-bendable wall section of the catheter of Fig. 1, wherein the shown view is a top view on the longitudinal section in an imaginary "unrolled tube" state of the longitudinal section.
Fig. 5A again shows a short longitudinal section of the pre-bendable skeleton of the pre-bendable wall section of the catheter of Fig. 1, however, wherein this time the shown view is a longitudinal side view.
Fig. 5B shows the longitudinal side view of Fig. 5A again, however, this time in a bent state of the longitudinal section as a result of a physician having pre-bent the pre-bendable wall section of the catheter of Fig. 1.
Fig. 6 shows, in a side view similar to the side view of Fig. 5A, an example of a distal skeleton of a distal section of a tubular catheter of a catheter according to the invention, wherein the distal skeleton of Fig. 6 is an end-to-end interconnection of the pre-bendable skeleton of Figs. 1-5 with a second skeleton and a third skeleton.
Figs. 7A-7E show details of the second skeleton of Fig. 6.
Figs. 8A-8C show details of the third skeleton of Fig. 6.
Fig. 9 shows, in a view similar to the view of Fig. 4, a short longitudinal section of an example of an alternative embodiment of a pre-bendable skeleton of a catheter according to the invention, wherein in the alternative embodiment of the pre-bendable skeleton of Fig. 9 the above-mentioned number N is equal to three (N = 3).

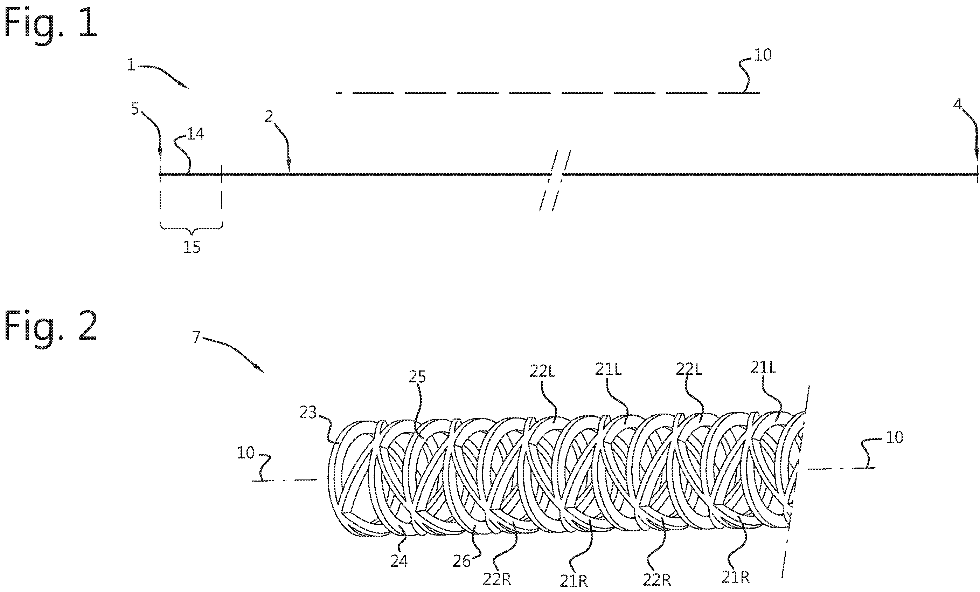

The reference signs used in the embodiment of Figs. 1-8 are referring to the above-mentioned parts of the invention, as well as to related parts, in the following manner.
- 1: catheter
- 2: tubular catheter wall
- 3: lumen structure
- 4: proximal end
- 5: distal end
- 6: distal skeleton
- 7: pre-bendable skeleton
- 7A: cut pattern
- 8: inner liner
- 9: outer laminate
- 10: longitudinal direction
- 11: circumferential direction
- 12: center line
- 14: pre-bendable wall section
- 15: pre-bending range
- 16: second range
- 17: third range
- 21L, 22L: two left-handed helical struts
- 21R, 22R: two right-handed helical struts
- 23-26: four ring-shaped struts
- 44: second skeleton
- 45: third skeleton
- 51-54: helical parts
- 61-66: helical slots
- 90-99: ten connection nodes

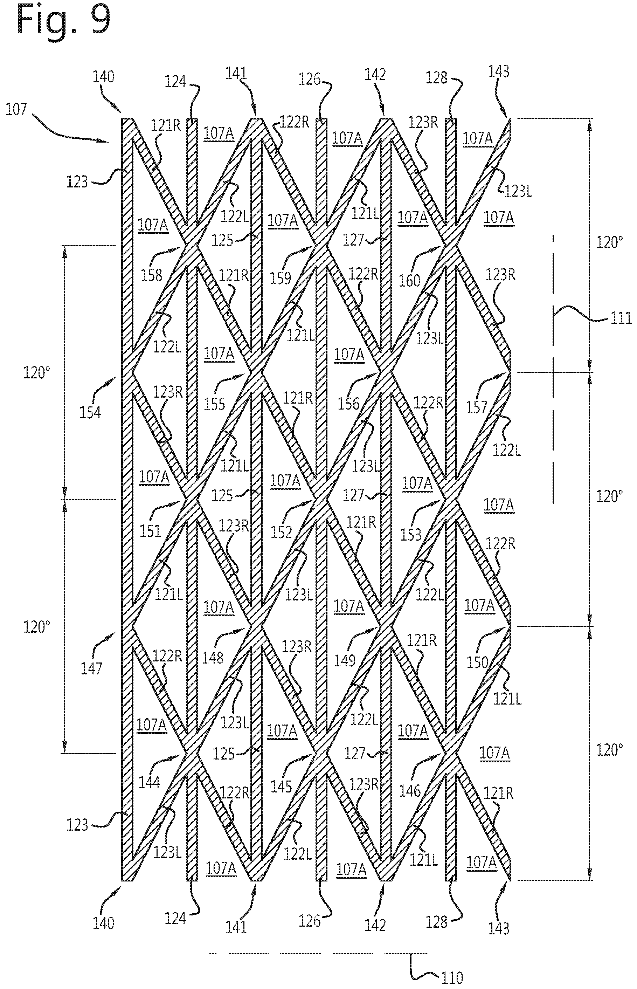

The reference signs used in the alternative embodiment of Fig. 9 are referring to the above-mentioned parts of the invention, as well as to related parts, in the following manner.
- 107: pre-bendable skeleton
- 107A: cut pattern
- 110: longitudinal direction
- 111: circumferential direction
- 121L, 122L, 123L: three left-handed helical struts
- 121R, 122R, 123R: three right-handed helical struts
- 123-128: six ring-shaped struts
- 140-160: twenty one connection nodes

Based on the above introductory description, including the brief description of the drawing figures, and based on the above-listed reference signs used in Figs. 1-9, the embodiment of Figs. 1-8 and the alternative embodiment of Fig. 9 are for the greatest part readily self-explanatory. The following extra explanations are given.

Fig. 1 shows the catheter 1 having the longitudinal direction 10, the proximal end 4 and the distal end 5. Fig. 1 further shows the pre-bending range 15 of the pre-bendable wall section 14 of the catheter wall 2.

In the embodiment of Figs. 1-8 the above-mentioned number N is equal to two (N = 2). This means that in the embodiment of Figs. 1-8 the pre-bendable skeleton 7 comprises two right-handed helical struts which are angularly equally spaced relative to one another by 180 degrees and two left-handed helical struts which are angularly equally spaced relative to one another by 180 degrees.

The perspective view of Fig. 2 shows that the pre-bendable skeleton 7 of the pre-bendable wall section 14 of the catheter 1 of Fig. 1 has the two left-handed helical struts 21L, 22L, the two right-handed helical struts 21R, 22R, and at least the mutually parallel ring-shaped struts 23-26.

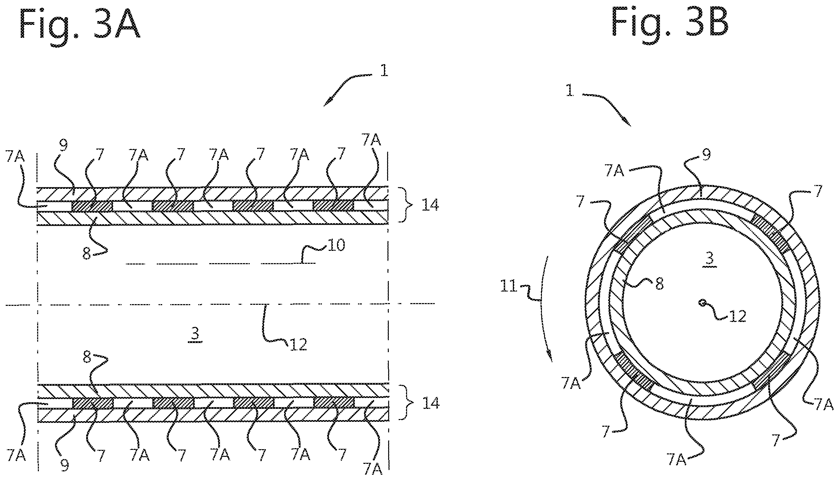

Figs. 3A-3B show that the pre-bendable wall section 14 not only has the skeleton 7, which comprises the cut pattern 7A, but also has the inner liner 8 and the outer laminate 9. In the example of Figs. 3A-3B, the lumen structure 3 of the catheter 1 is a single lumen structure. Alternatively, a catheter according to the invention may have a multiple lumen structure.

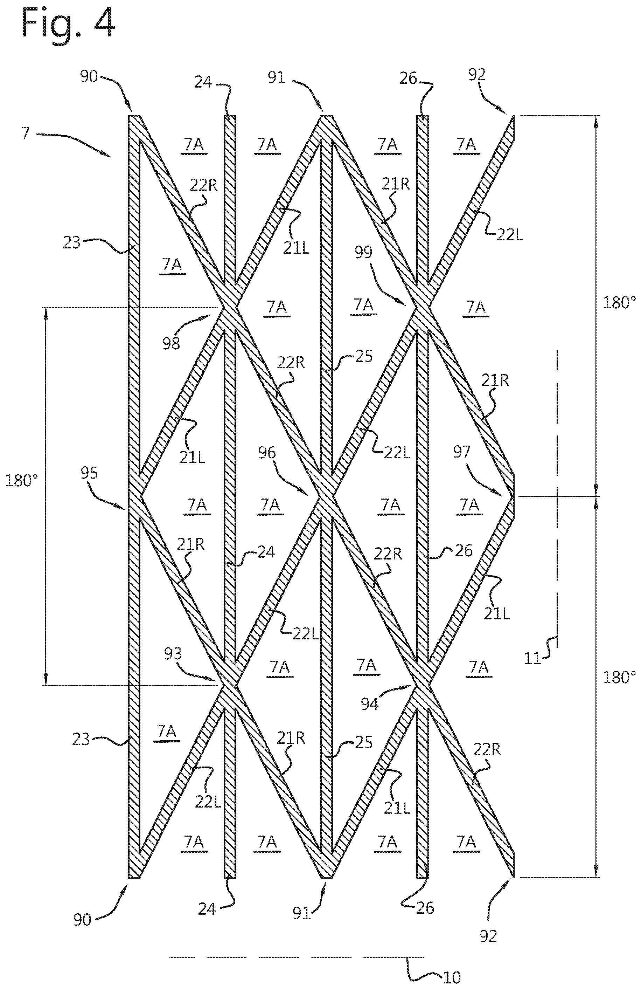

Fig. 4 shows a short longitudinal section of the pre-bendable skeleton 7 of the pre-bendable wall section 14 of the catheter 1. In Fig. 4, the shown view is a top view on the longitudinal section in an imaginary "unrolled tube" state of the longitudinal section. To explain the term "unrolled tube" it is noted that the pre-bendable skeleton is a tubular wall (more particularly an interrupted tubular reinforcement wall), and that such a tubular wall imaginarily can be unrolled into a completely straight state by imaginarily unrolling it onto a fully straight plane after an imaginary straight cut, parallel to the center line of the catheter, has been made over the full length of the tubular wall. In Fig. 4 said imaginary straight cut is a straight line that passes through the connection nodes 90, 91 and 92 of the pre-bendable skeleton 7. In Fig. 4 the reference numerals 90, 91 and 92 are shown both in the lower part of Fig. 4 and in the upper part of Fig. 4, since the locations to which these reference numerals 90, 91 and 92 are pointing will join when the completely straight state of Fig. 4 would be "rolled up" again to form the tubular state of the pre-bending skeleton 7 as shown in the perspective view of Fig. 2.

From Fig. 4 it is seen that:
- the left-handed helical strut 22L is extending successively from the connection node 90, to the connection node 93, to the connection node 96 to the connection node 99, to the connection node 92; and
- the left-handed helical strut 21L is extending successively from the connection node 95, to the connection node 98, to the connection node 91 to the connection node 94, to the connection node 97;
- the right-handed helical strut 21R is extending successively from the connection node 95, to the connection node 93, to the connection node 91 to the connection node 99, to the connection node 97; and
- the right-handed helical strut 22R is extending successively from the connection node 90, to the connection node 98, to the connection node 96 to the connection node 94, to the connection node 92.

From Fig. 4 it can be inferred that the two right-handed helical struts 21R and 22R are angularly spaced relative to one another by 180 degrees around the center line 12, that the two left-handed helical struts 21L and 22L are angularly spaced relative to one another by 180 degrees around the center line 12, and that the ring-shaped struts 23, 24, 25, 26 are extending mutually parallel relative to one another and mutually spaced apart in the longitudinal direction 10.

From Fig. 4 it can further be inferred that the pre-bendable skeleton 7 comprises for each of the shown ring-shaped struts 23, 24, 25, 26 two connection nodes at two diametrically opposite positions, respectively, of each ring-shaped strut concerned, and that at each connection node, each ring-shaped strut concerned is connected to one of the two right-handed helical struts 21R, 22R as well as to one of the two left-handed helical struts 21L, 22L.

Fig. 4 clearly illustrates that the pre-bendable skeleton 7 is a triangular structure formed by nothing but triangles. That is, all connection nodes 90-99 of the pre-bendable skeleton 7 are vertices of multiple triangles, in which each triangle is formed by three strut sections of a left-handed helical strut, a right-handed helical strut and a ring-shaped strut, respectively. Such a triangular structure is strong and allows for favourable shape retention.

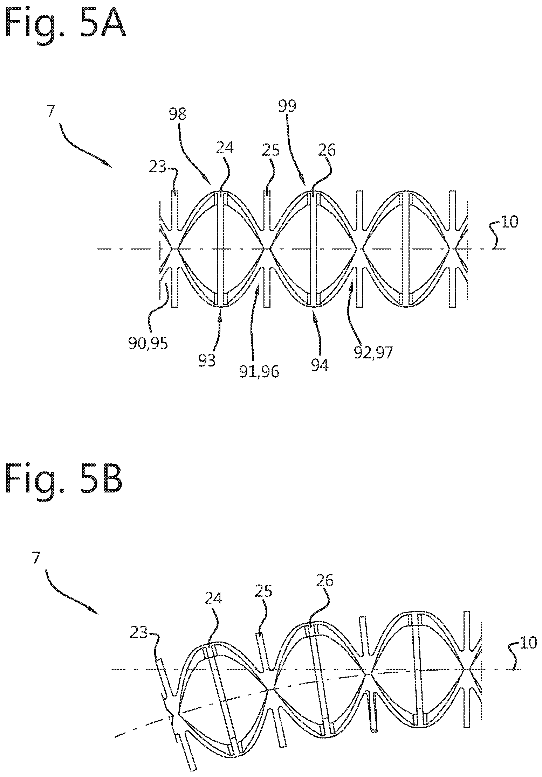

Figs. 5A and 5B illustrate that the triangular structure of the pre-bendable skeleton 7 will only be slightly deformed when a physician has pre-bent the pre-bendable wall section 14 of the catheter 1. The parallel ring-shaped struts (including the shown ring-shaped struts 23-26), provide hoop strength without hampering the pre-bendability of the pre-bendable wall section. At the same time said pre-bendability is guided and regulated by the positions of the connection nodes (including the shown connection nodes 90-99), as well as by the parallel ring-shaped struts. Clearly, the multi-triangular character of the pre-bendable skeleton 7 remains intact after pre-bending, and so does the skeleton's good shape retention.

In the shown example the pre-bendable skeleton 7 has the following dimensions. The pre-bending range 15 is extending between the distal end 5 and 20.0 mm proximal from the distal end 5. So the pre-bendable skeleton 7 has a length of 20.0 mm in the longitudinal direction 10. The pre-bendable skeleton 7 has an outer diameter of 0.635 mm and an inner diameter of 0.510 mm. The pitch of each of the two right-handed helical struts 21R, 22R and the two left-handed helical struts 21L, 22L is 1.00 mm, wherein the pitch is defined as the distance, measured along the longitudinal direction 10, of one helical winding of a helical strut concerned. So in Fig. 4 the pitch of each of the helical struts is equal to the distance, measured along the longitudinal direction 10, between the two centers of the two connection nodes 90 and 92, respectively. The mutually parallel ring-shaped struts are spaced apart relative to one another over 0.250 mm as measured along the longitudinal direction 10. The width of each of the helical struts and ring-shaped struts is 0.030 mm as measured in the "unrolled tube" state of the pre-bendable skeleton 7 shown in Fig. 4. The overall skeleton cut-away ratio of the pre-bendable wall section 14 in the pre-bending range 15 is about 76%.

It is noted that in the shown example, the pre-bendable skeleton 7 ends at the distal end 5 of the catheter 1. Alternatively, however, the inner liner 8 and/or the outer laminate 9 may extend a bit distally beyond the pre-bendable skeleton 7 to make the distal end 5 atraumatic.

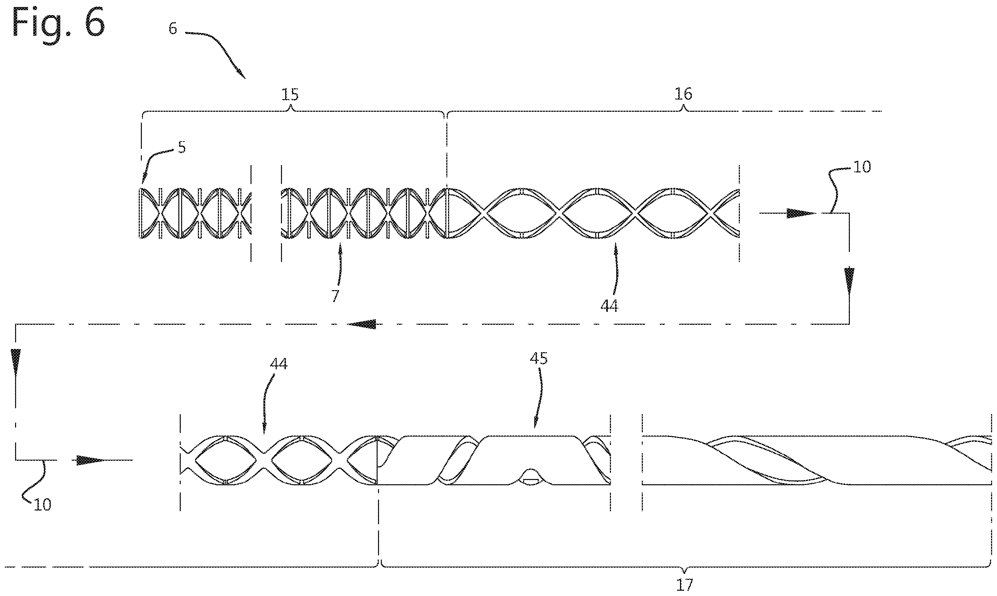

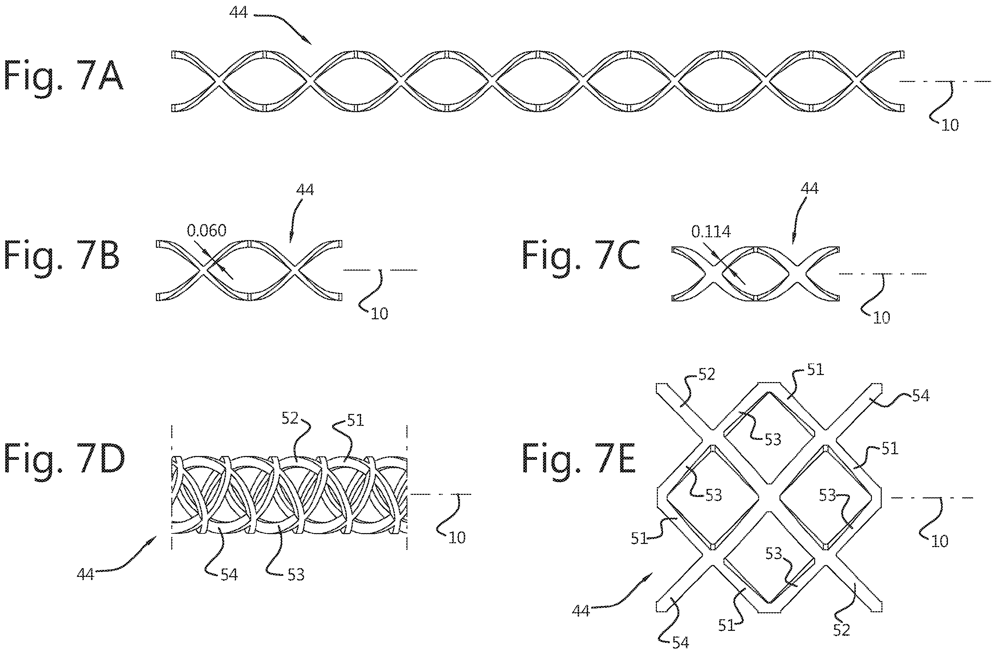

Fig. 6 shows the pre-bendable skeleton 7 in the pre-bending range 15 together with a second skeleton 44 and a third skeleton 45. The three skeletons 7, 44 and 45 are successively interconnected in an end-to-end manner along the longitudinal direction 10 to form a distal skeleton 6 of a distal section of the tubular catheter wall 2. The second skeleton 44 is extending in a second range 16, which is extending in the longitudinal direction 10 from 20.0 mm proximal from the distal end 5 to 100.0 mm proximal from the distal end 5. The third skeleton 45 is extending in a third range 17, which is extending in the longitudinal direction 10 from 100.0 mm proximal from the distal end 5 to 150.0 mm proximal from the distal end 5. The second skeleton 44 and the third skeleton 45 are shown in more detail in Figs. 7-8, respectively.

Reference is now made to Figs. 7A-7E, which illustrate the second skeleton 44. The second skeleton 44 is extending in the second range 16 (see Fig. 6) along the longitudinal direction 10 over a length of 80.0 mm. Each of Figs. 7A-7E shows the second skeleton 44 only along a part of the last-mentioned length. Fig. 7A shows a part of the second skeleton 44 in longitudinal side view. Figs. 7B and 7C show two other parts of the second skeleton 44 in longitudinal side view, wherein the part of Fig. 7B is located distally from the part of Fig. 7A, and the part of Fig. 7C is located proximally from the part of Fig. 7A. Fig. 7D is a perspective view of the second skeleton 44. Fig. 7E shows a top view on a longitudinal section of the second skeleton 44 in an imaginary unrolled tube state of the longitudinal section (the term "unrolled tube" has been explained above with reference to Fig. 4). Figs. 7D-7E show that the second skeleton 44 consists of four helical parts, two of which being right-handed helical parts 51, 52 and two of which being left-handed helical parts 53, 54, wherein the two right-handed helical parts 51, 52 are intersecting the two left-handed helical parts 53, 54 at multiple nodes of the second skeleton 44.

In Figs. 7B-7C some dimensions have been indicated by numerical values, which are to be interpreted in millimeters (mm). Figs. 7B-7C serve to illustrate the aspect that the thickness of the helical parts of the second skeleton 44 gradually decreases distally along the longitudinal direction. Thereby, the flexibility of the second skeleton 44 of the catheter wall 2 gradually increases distally along the longitudinal direction 10.

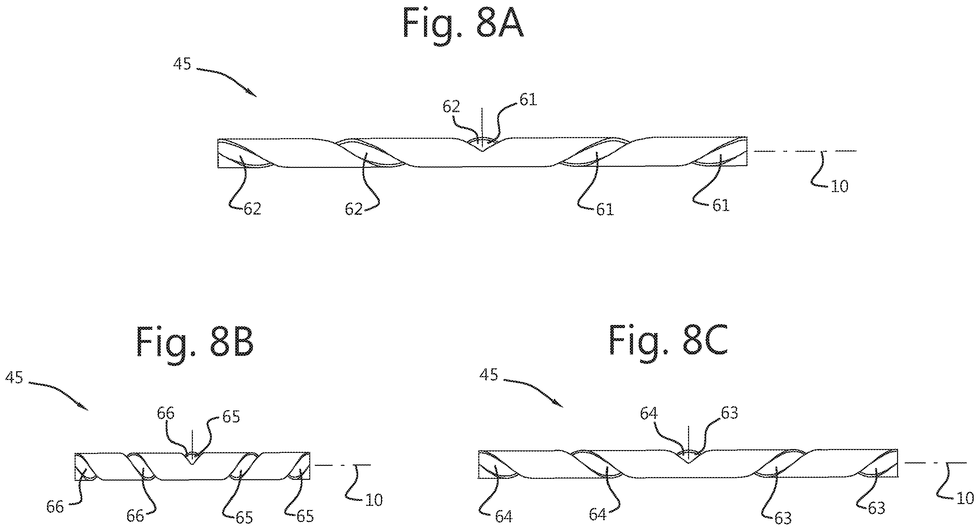

Reference is now made to Figs. 8A-8C, which show the third skeleton 45 in longitudinal side view. The third skeleton 45 is extending in the third range 17 (see Fig. 6) along the longitudinal direction 10 over a length of 50.0 mm. Each of Figs. 8A-8C shows the third skeleton 45 only along a part of the last-mentioned length. The part of Fig 8C is located distally away from the part of Fig. 8A. The part of Fig. 8B is located distally away from the part of Fig. 8C. The third skeleton 45 has a number of helical slots which are interconnected in an end-to-end manner along the longitudinal direction 10. Each helical slot has only a few helical windings. For example, Fig. 8A shows the helical slots 61 and 62, Fig. 8C shows the helical slots 63 and 64, and Fig. 8B shows the helical slots 65 and 66. Two mutually connected ones of the helical slots each time have opposite winding directions relative to one another. Such mutual connections of two oppositely directed helical slots are shown in the longitudinal middle of each of the Figs. 8A-8C. Figs. 8A-8C further serve to illustrate that the pitch of the helical slots gradually decreases for distally subsequent helical slots along the longitudinal direction 10. Due to said decreasing pitch, the overall skeleton cut-away ratio of the catheter wall 2 in the third range 17 gradually increases distally, with the effect that the flexibility of the catheter wall 2 in the third range 17 gradually increases distally along the longitudinal direction.

The three interconnected skeletons 7, 44 and 45 of the distal skeleton 6 of Fig. 6 can be integrally manufactured by microfabricating a cut pattern in a fully uninterrupted tubular reinforcement wall made of a reinforcement material.

A tubular catheter wall which comprises the pre-bendable skeleton 7 or the larger distal skeleton 6 may for example be part of a rapid exchange catheter having the tubular catheter wall as a distal rapid exchange tubular segment which is spaced from the proximal end of the rapid exchange catheter. Alternatively, such a tubular catheter wall may for example be part of an over-the-wire (OTW) catheter, wherein the tubular catheter wall is extending all along the longitudinal direction from the proximal end to the distal end.

As mentioned, the embodiment of Figs. 1-8 is an embodiment in which N = 2. It is further noted that the embodiment of Figs. 1-8 more specifically is an example of the above-mentioned preferable embodiment of a catheter according to the invention, wherein the N (i.e. two) right-handed helical struts (21R, 22R) and the N (i.e. two) left-handed helical struts (21L, 22L) each have the same pitch as compared to one another, said pitch being defined as the distance, measured along the longitudinal direction, of one helical winding of a helical strut concerned, and wherein the pre-bending range has at least one continuous sub-range having a length equal to said pitch, and wherein the pre-bendable skeleton has a number of N multiplied by 2 (i.e. four) of said ring-shaped struts (23, 24, 25, 26) in said continuous sub-range. This is clear from Fig. 4, which is a short longitudinal section of the pre-bendable skeleton 7, wherein said short longitudinal section corresponds to such a continuous sub-range having a length equal to said pitch. In Fig. 4 it is seen that the pre-bendable skeleton 7 has ten connection nodes (90-99) in said continuous sub-range.

Reference is now made to Fig. 9, which shows the pre-bendable skeleton 107 as an alternative embodiment to the pre-bendable skeleton 7 of Fig. 4. In the alternative pre-bendable skeleton 107 of Fig. 9 the above-mentioned number N is equal to three (N = 3). This means that in the embodiment of Fig. 9 the pre-bendable skeleton 107 comprises N (i.e. three) right-handed helical struts (121R, 122R, 123R) which are angularly equally spaced relative to one another by 120 degrees and N (i.e. three) left-handed helical struts (121L, 122L, 123L) which are angularly equally spaced relative to one another by 120 degrees.

It is further noted that, when the alternative pre-bendable skeleton 107 is used in the catheter embodiment of Figs. 1-8 to replace the pre-bendable skeleton 7, there is more specifically provided an example of the above-mentioned preferable embodiment of a catheter according to the invention, wherein the N (i.e. three) right-handed helical struts and the N (i.e. three) left-handed helical struts each have the same pitch as compared to one another, said pitch being defined as the distance, measured along the longitudinal direction, of one helical winding of a helical strut concerned, and wherein the pre-bending range has at least one continuous sub-range having a length equal to said pitch, and wherein the pre-bendable skeleton has a number of N multiplied by 2 (i.e. six) of said ring-shaped struts (123, 124, 125, 126, 127, 128) in said continuous sub-range. This is clear from Fig. 9, which is a short longitudinal section of the pre-bendable skeleton 107, wherein said short longitudinal section corresponds to such a continuous sub-range having a length equal to said pitch. In Fig. 9 it is seen that the pre-bendable skeleton 107 has twenty one connection nodes (140-160) in said continuous sub-range.

From the above-explained example embodiments of a catheter according to the invention for N = 2 and N = 3, respectively, it will be sufficiently clear to the reader of the present disclosure how examples of embodiments of a catheter according to the invention for N ≥ 4 can be designed analogously.

The invention can be practiced with many various structures, many various materials, many various shapes and many various dimensions of the pre-bendable skeleton, inner liner and outer laminate of the catheter, and with many various additional features and many various accessories of the catheter, such as the many various structures, the many various materials, the many various shapes and the many various dimensions of skeletons, inner liners and outer laminates, and the many various additional features and the many various accessories that are used in known catheters for delivering medical therapy to a remote site in a body, as long as the catheter is within the scope of the appended claims.

Regarding the overall skeleton cut-away ratio the following general remark is made. If, everywhere in a longitudinally continuous longitudinal section of a catheter wall, the catheter wall has a totally uninterrupted tubular metal reinforcement wall, for example in the form of a totally uninterrupted metal hypotube, then said longitudinal section has an overall skeleton cut-away ratio of 0%.

It is further noted that according to the invention typical dimensions of some parts of the invention, and applicable practical ranges of such dimensions could be as follows in case the catheter is a microcatheter for interventional cardiovascular or neurovascular applications. Effective length of the catheter (as measured from the distal end of a hub of the catheter) can be in the range between 130 cm and 160 cm, and can typically be 135 cm. Maximum outer diameter of the pre-bendable wall section of the tubular catheter wall of the catheter can be in the range between 0.60 mm and 1.20 mm, and can typically be 0.75 mm, while noting that the word "maximum" in the term "maximum outer diameter" is referring to the maximum value as considered over the full length of the pre-bendable wall section. Minimum inner diameter of the pre-bendable wall section of the tubular catheter wall of the catheter can be in the range between 0.36 mm and 0.55 mm, and can typically be 0.45 mm, while noting that the word "minimum" in the term "minimum inner diameter" is referring to the minimum value as considered over the full length of the pre-bendable wall section. For example, the following typical combinations of maximum outer diameter and minimum inner diameter of the pre-bendable wall section are possible: maximum outer diameter 0.75 mm in combination with minimum inner diameter 0.45 mm, maximum outer diameter 1.20 mm in combination with minimum inner diameter 0.55 mm, or maximum outer diameter 0.60 mm in combination with minimum inner diameter 0.36 mm.

However, the present invention can be embodied in various other catheters for delivering medical therapy to a remote site in a body, which can be non-vascular and/or non-micro catheters, such as for example catheters for delivering medical therapy to renal vessels, fallopian tubes, and other such vessels and sites.

## Claims

1. A catheter (1) for delivering medical therapy to a remote site in a body, wherein:
the catheter has a proximal end (4), a distal end (5) and a longitudinal direction (10) extending from the proximal end to the distal end;
the catheter comprises a tubular catheter wall (2), which is surrounding a lumen structure (3) of the catheter, and which is extending from the distal end (5), proximally along the longitudinal direction, to at least 100 mm proximal from the distal end;
the catheter wall (2) comprises a pre-bendable wall section (14), which is manually pre-bendable all along a pre-bending range (15) along the longitudinal direction, wherein said pre-bending range is at least extending between 5 mm proximal from the distal end and 10 mm proximal from the distal end (5);
the pre-bendable wall section (14) comprises:
- a pre-bendable skeleton (7), which is extending at least all along said pre-bending range, and which provides reinforcement of the catheter wall (2) in the longitudinal direction (10), as well in a circumferential direction (11) around the longitudinal direction (10), wherein the pre-bendable skeleton (7) is an interrupted tubular reinforcement wall obtained by microfabricating a cut pattern (7A) in a fully uninterrupted tubular reinforcement wall made of a reinforcement material,
- an inner liner (8), which is co-axially surrounded by said pre-bendable skeleton (7) relative to a center line (12) of the catheter wall (2), and
- an outer laminate (9), which is co-axially surrounding said pre-bendable skeleton (7) relative to the center line (12) of the catheter wall;
the pre-bendable skeleton comprises:
- a number N of right-handed helical struts (21R, 22R), which are extending along N right-handed helical directions, respectively, wherein N ≥ 2, and
- N left-handed helical struts (21L, 22L), which are extending along N left-handed helical directions, respectively;
**characterized in that**:
the pre-bendable skeleton further comprises a plurality of ring-shaped struts (23-26), which are extending along circular directions, respectively;
each of said right-handed helical struts (21R, 22R), left-handed helical struts (21L, 22L) and ring-shaped struts (23-26) are co-axially arranged relative to the center line (12), wherein the N right-handed helical struts are angularly equally spaced relative to one another by 360 divided by N degrees around the center line (12), wherein the N left-handed helical struts are angularly equally spaced relative to one another by 360 divided by N degrees around the center line, wherein the ring-shaped struts are extending mutually parallel relative to one another and mutually spaced apart in the longitudinal direction, wherein the pre-bendable skeleton (7) comprises for each of said ring-shaped struts N connection nodes (90-99) at N angularly equally spaced positions, respectively, of each ring-shaped strut concerned, and wherein at each connection node (90-99), each ring-shaped strut (23-26) concerned is connected to one of the N right-handed helical struts (21R, 22R) as well as to one of the N left-handed helical struts (21L, 22L);
an overall skeleton cut-away ratio of a longitudinal section of the catheter wall (2) in a longitudinal range along the longitudinal direction (10) is defined as the percentage of:
- the volume of all parts, within said longitudinal range, of said reinforcement material being cut-away from said fully uninterrupted tubular reinforcement wall according to said microfabricated cut pattern (7A), relative to
- the volume of all parts, within said longitudinal range, of said reinforcement material of said fully uninterrupted tubular reinforcement wall; and
the overall skeleton cut-away ratio of the pre-bendable wall section (14) in said pre-bending range (15) is between 45% and 85%.

2. The catheter (1) according to claim 1, wherein said pre-bending range (15) is at least extending between 0.5 mm proximal from the distal end (5) and 15 mm proximal from the distal end (5).

3. The catheter (1) according to claim 2, wherein said pre-bending range is at least extending between the distal end (5) and 20 mm proximal from the distal end (5).

4. The catheter (1) according to any one of the preceding claims, wherein the overall skeleton cut-away ratio of the pre-bendable wall section (14) in said pre-bending range (15) is between 60% and 80%.

5. The catheter (1) according to claim 4, wherein the overall skeleton cut-away ratio of the pre-bendable wall section (14) in said pre-bending range (15) is between 70% and 78%.

6. The catheter (1) according to any one of the preceding claims, wherein a radiopaque element is embedded in the catheter wall (2) in that the radiopaque element is located in a space of the cut pattern (7A), said space being bounded by the pre-bendable skeleton (7), the inner layer (8) and the outer laminate (9).

7. The catheter (1) according to any one of the preceding claims, wherein the catheter is a rapid exchange catheter having the tubular catheter wall (2) as a distal rapid exchange tubular segment which is spaced from the proximal end (4) of the rapid exchange catheter.

8. The catheter (1) according to any one of claims 1-6, wherein the catheter is an over-the-wire (OTW) catheter, wherein the tubular catheter wall (2) and the lumen structure (3) of the catheter are extending all along the longitudinal direction (10) from the proximal end (4) to the distal end (5).

9. The catheter (1) according to any one of the preceding claims, wherein the N right-handed helical struts (21R, 22R) and the N left-handed helical struts (21L, 22L) each have the same pitch as compared to one another, said pitch being defined as the distance, measured along the longitudinal direction (10), of one helical winding of a helical strut concerned, and wherein the pre-bending range (15) has at least one continuous sub-range having a length equal to said pitch, and wherein the pre-bendable skeleton (7) has a number of N multiplied by 2 of said ring-shaped struts in said continuous sub-range.

## Patentansprüche

1. Katheter (1) zur Verabreichung einer medizinischen Therapie an eine entfernte Stelle in einem Körper, wobei:
der Katheter ein proximales Ende (4), ein distales Ende (5) und eine Längsrichtung (10) aufweist, die sich vom proximalen Ende zum distalen Ende erstreckt;
der Katheter eine röhrenförmige Katheterwand (2) umfasst, die eine Lumenstruktur (3) des Katheters umgibt und die sich vom distalen Ende (5), proximal entlang der Längsrichtung, bis mindestens 100 mm proximal vom distalen Ende erstreckt;
die Katheterwand (2) einen vorbiegbaren Wandabschnitt (14) umfasst, der entlang eines gesamten Vorbiegebereichs (15) entlang der Längsrichtung manuell vorbiegbar ist, wobei sich der Vorbiegebereich mindestens zwischen 5 mm proximal vom distalen Ende und 10 mm proximal vom distalen Ende (5) erstreckt;
der vorbiegbare Wandabschnitt (14) umfasst:
- ein vorbiegbares Skelett (7), das sich wenigstens entlang des gesamten Vorbiegebereichs erstreckt und das eine Verstärkung der Katheterwand (2) in der Längsrichtung (10) sowie in einer Umfangsrichtung (11) um die Längsrichtung (10) bereitstellt, wobei das vorbiegbare Skelett (7) eine unterbrochene röhrenförmige Verstärkungswand ist, die durch Mikrofertigung eines Schnittmusters (7A) in einer vollständig ununterbrochenen röhrenförmigen Verstärkungswand, hergestellt aus einem Verstärkungsmaterial, erhalten wird,
- eine Innenauskleidung (8), die koaxial von dem vorbiegbaren Skelett (7) relativ zu einer Mittellinie (12) der Katheterwand (2) umgeben ist, und
- ein äußeres Laminat (9), das das vorbiegbare Skelett (7) relativ zur Mittellinie (12) der Katheterwand koaxial umgibt;
das vorbiegbare Skelett umfasst:
- eine Anzahl N von rechtsdrehenden Spiralstreben (21R, 22R), die sich jeweils entlang N rechtsdrehender Spiralrichtungen erstrecken, wobei N ≥ 2 und
- N linksdrehende Spiralstreben (21L, 22L), die sich jeweils entlang N linksdrehender Spiralrichtungen erstrecken;
**dadurch gekennzeichnet, dass**:
das vorbiegbare Skelett ferner eine Mehrzahl an ringförmigen Streben (23-26) umfasst, die sich jeweils entlang kreisförmiger Richtungen erstrecken;
jede der rechtsdrehenden Spiralstreben (21R, 22R), linksdrehenden Spiralstreben (21L, 22L) und ringförmigen Streben (23-26) relativ zur Mittellinie (12) koaxial angeordnet ist, wobei die N rechtsdrehenden Spiralstreben relativ zueinander um 360 geteilt durch N Grad um die Mittellinie (12) winkelgleich beabstandet sind, wobei die N linksdrehenden Spiralstreben relativ zueinander um 360 geteilt durch N Grad um die Mittellinie beabstandet sind, wobei sich die ringförmigen Streben parallel zueinander erstrecken und in Längsrichtung voneinander beabstandet sind, wobei das vorbiegbare Skelett (7) für jede der ringförmigen Streben N Verbindungsknoten (90-99) an N jeweils winkelgleich beabstandeten Positionen jeder betreffenden ringförmigen Strebe umfasst, und wobei an jedem Verbindungsknoten (90-99) jede betreffende ringförmige Strebe (23-26) mit einer der N rechtsdrehenden Spiralstreben (21R, 22R) sowie mit einer der N linksdrehenden Spiralstreben (21L, 22L) verbunden ist;
ein Gesamtskelettschnittverhältnis eines Längsabschnitts der Katheterwand (2) in einem Längsbereich entlang der Längsrichtung (10) definiert ist als der Prozentsatz:
- des Volumens aller Teile, innerhalb des Längsbereichs, des Verstärkungsmaterials, das von der vollständig ununterbrochenen röhrenförmigen Verstärkungswand gemäß dem mikrogefertigten Schnittmuster (7A) abgeschnitten ist, relativ zu
- des Volumens aller Teile, innerhalb des Längsbereichs, des Verstärkungsmaterials der vollständig ununterbrochenen röhrenförmigen Verstärkungswand; und
das Gesamtskelettschnittverhältnis des vorbiegbaren Wandabschnitts (14) in dem Vorbiegebereich (15) zwischen 45 % und 85 % liegt.

2. Katheter (1) nach Anspruch 1, wobei sich der Vorbiegebereich (15) wenigstens zwischen 0,5 mm proximal vom distalen Ende (5) und 15 mm proximal vom distalen Ende (5) erstreckt.

3. Katheter (1) nach Anspruch 2, wobei sich der Vorbiegebereich wenigstens zwischen dem distalen Ende (5) und 20 mm proximal vom distalen Ende (5) erstreckt.

4. Katheter (1) nach einem der vorhergehenden Ansprüche, wobei das Gesamtskelettschnittverhältnis des vorbiegbaren Wandabschnitts (14) in dem Vorbiegebereich (15) zwischen 60 % und 80 % liegt.

5. Katheter (1) nach Anspruch 4, wobei das
Gesamtskelettschnittverhältnis des vorbiegbaren Wandabschnitts (14) in dem Vorbiegebereich (15) zwischen 70 % und 78 % liegt.

6. Katheter (1) nach einem der vorhergehenden Ansprüche, wobei ein strahlendichtes Element insofern in die Katheterwand (2) eingebettet ist, als sich das strahlendichte Element in einem Raum des Schnittmusters (7A) befindet, wobei dieser Raum vom vorbiegbaren Skelett (7), der inneren Schicht (8) und dem äußeren Laminat (9) begrenzt ist.

7. Katheter (1) nach einem der vorhergehenden Ansprüche, wobei es sich bei dem Katheter um einen Schnellwechselkatheter handelt, der die röhrenförmige Katheterwand (2) als ein distales Schnellwechselröhrensegment aufweist, das vom proximalen Ende (4) des Schnellwechselkatheters beabstandet ist.

8. Katheter (1) nach einem der Ansprüche 1 bis 6, wobei der Katheter ein Over-the-Wire-(OTW-)Katheter ist, wobei sich die röhrenförmige Katheterwand (2) und die Lumenstruktur (3) des Katheters entlang der gesamten Längsrichtung (10) vom proximalen Ende (4) zum distalen Ende (5) erstrecken.

9. Katheter (1) nach einem der vorhergehenden Ansprüche, wobei die N rechtsdrehenden Spiralstreben (21R, 22R) und die N linksdrehenden Spiralstreben (21L, 22L) jeweils den gleiche Abstand zueinander aufweisen, wobei der Abstand definiert ist als die Entfernung, gemessen entlang der Längsrichtung (10), einer Spiralwindung einer betroffenen Spiralstrebe, und wobei der Vorbiegebereich (15) wenigstens einen kontinuierlichen Teilbereich aufweist, der eine dem Abstand gleiche Länge aufweist, und wobei das vorbiegbare Skelett (7) eine Anzahl von N multipliziert mit 2 der ringförmigen Streben in dem durchgehenden Teilbereich aufweist.

## Revendications

1. Cathéter (1) pour administrer une thérapie médicale sur un site distant dans un corps, dans lequel :
le cathéter a une extrémité proximale (4), une extrémité distale (5) et une direction longitudinale (10) s'étendant de l'extrémité proximale à l'extrémité distale ;
le cathéter comprend une paroi de cathéter tubulaire (2) qui entoure une structure de lumière (3) du cathéter, et qui s'étend à partir de l'extrémité distale (5), de manière proximale le long de la direction longitudinale, jusqu'à au moins 100 mm à proximité de l'extrémité distale ;
la paroi de cathéter (2) comprend une section de paroi pré-cintrable (14) qui est manuellement pré-cintrable sur toute une plage de pré-cintrage (15) le long de la direction longitudinale, dans lequel ladite plage de pré-cintrage s'étend au moins entre 5 mm à proximité de l'extrémité distale et 10 mm à proximité de l'extrémité distale (5) ;
la section de paroi pré-cintrable (14) comprend :
- un squelette pré-cintrable (7) qui s'étend au moins sur toute ladite plage de pré-cintrage, et qui fournit le renforcement de la paroi de cathéter (2) dans la direction longitudinale (10), ainsi que dans une direction circonférentielle (11) autour de la direction longitudinale (10), dans lequel le squelette pré-cintrable (7) est une paroi de renforcement tubulaire interrompue obtenue par micro-fabrication d'un motif de découpe (7A) dans une paroi de renforcement tubulaire complètement ininterrompue réalisée avec un matériau de renforcement,
- un revêtement interne (8) qui est entouré, de manière coaxiale, par ledit squelette pré-cintrable (7) par rapport à une ligne centrale (12) de la paroi de cathéter (2), et
- un stratifié externe (9), qui entoure, de manière coaxiale, ledit squelette pré-cintrable (7) par rapport à la ligne centrale (12) de la paroi de cathéter ;
le squelette pré-cintrable comprend :
- un nombre N d'entretoises hélicoïdales droites (21R, 22R) qui s'étendent respectivement le long de N directions hélicoïdales droites, dans lequel N ≥ 2, et
- N entretoises hélicoïdales gauches (21L, 22L), qui s'étendent respectivement le long de N directions hélicoïdales gauches ;
**caractérisé en ce que** :
le squelette pré-cintrable comprend en outre une pluralité d'entretoises de forme annulaire (23-26) qui s'étendent respectivement le long des directions circulaires ;
chacune desdites entretoises hélicoïdales droites (21R, 22R), des entretoises hélicoïdales gauches (21L, 22L) et des entretoises de forme annulaire (23-26) sont agencées, de manière coaxiale, par rapport à la ligne centrale (12), dans lequel les N entretoises hélicoïdales droites sont espacées à égale distance angulaire les unes par rapport aux autres de 360 divisés par N degrés autour de la ligne centrale (12), dans lequel les N entretoises hélicoïdales gauches sont espacées à égale distance angulaire les unes des autres de 360 divisés par N degrés autour de la ligne centrale, dans lequel les entretoises de forme annulaire s'étendent de manière mutuellement parallèle les unes par rapport aux autres et sont mutuellement espacées dans la direction longitudinale, dans lequel le squelette pré-cintrable (7) comprend pour chacune desdites entretoises de forme annulaire, N nœuds de connexion (90-99) respectivement dans N positions espacées à égale distance angulaire, de chaque entretoise de forme annulaire concernée, et dans lequel à chaque nœud de connexion (90-99), chaque entretoise de forme annulaire (23-26) concernée est raccordée à l'une parmi les N entretoises hélicoïdales droites (21R, 22R) ainsi que l'une parmi les N entretoises hélicoïdales gauches (21L, 22L) ;
un rapport global de découpe de squelette d'une section longitudinale de la paroi de cathéter (2) dans une plage longitudinale le long de la direction longitudinale (10) est défini comme étant le pourcentage de :
- le volume de toutes les parties, dans ladite plage longitudinale, dudit matériau de renforcement découpé dans ladite paroi de renforcement tubulaire complètement ininterrompue selon ledit motif de découpe micro-fabriqué (7A),
par rapport au
- volume de toutes les parties, dans ladite plage longitudinale, dudit matériau de renforcement de ladite paroi de renforcement tubulaire complètement ininterrompue ; et
le rapport global de découpe de squelette de la section de paroi pré-cintrable (14) dans ladite plage de pré-cintrage (15) est compris entre 45 % et 85 %.

2. Cathéter (1) selon la revendication 1, dans lequel ladite plage de pré-cintrage (15) s'étend au moins entre 0,5 mm à proximité de l'extrémité distale (5) et 15 mm à proximité de l'extrémité distale (5).

3. Cathéter (1) selon la revendication 2, dans lequel ladite plage de pré-cintrage s'étend au moins entre l'extrémité distale (5) et 20 mm à proximité de l'extrémité distale (5).

4. Cathéter (1) selon l'une quelconque des revendications précédentes, dans lequel le rapport global de découpe de squelette de la section de paroi pré-cintrable (14) dans ladite plage de pré-cintrage (15) est compris entre 60 % et 80 %.

5. Cathéter (1) selon la revendication 4, dans lequel le rapport global de découpe de squelette de la section de paroi pré-cintrable (14) dans ladite plage de pré-cintrage (15) est compris entre 70 % et 78 %.

6. Cathéter (1) selon l'une quelconque des revendications précédentes, dans lequel un élément radio-opaque est encastré dans la paroi de cathéter (2), en ce que l'élément radio-opaque est situé dans un espace du motif de découpe (7A), ledit espace étant délimité par le squelette pré-cintrable (7), la couche interne (8) et le stratifié externe (9).

7. Cathéter (1) selon l'une quelconque des revendications précédentes, dans lequel le cathéter est un cathéter à échange rapide ayant la paroi de cathéter tubulaire (2) en tant que segment tubulaire distal à échange rapide qui est espacé de l'extrémité proximale (4) du cathéter à échange rapide.

8. Cathéter (1) selon l'une quelconque des revendications 1 à 6, dans lequel le cathéter est un cathéter sur le fil (OTW), dans lequel la paroi de cathéter tubulaire (2) et la structure de lumière (3) du cathéter s'étendent sur toute la direction longitudinale (10) de l'extrémité proximale (4) à l'extrémité distale (5).

9. Cathéter (1) selon l'une quelconque des revendications précédentes, dans lequel les N entretoises hélicoïdales droites (21R,22R) et les N entretoises hélicoïdales gauches (21L, 22L) ont le même pas les unes par rapport aux autres, ledit pas étant défini comme étant la distance mesurée le long de la direction longitudinale (10), d'un enroulement hélicoïdal d'une entretoise hélicoïdale concernée, et dans lequel la plage de pré-cintrage (15) a au moins une sous-plage continue ayant une longueur égale audit pas, et dans lequel le squelette pré-cintrable (7) a un nombre N multiplié par 2 desdites entretoises de forme annulaire dans ladite sous-plage continue.
